# EUROPEAN PATENT APPLICATION

(11) **EP 4 049 669 A1**
(43) Date of publication of application: **31.08.2022**
(21) Application number: 21180393.7
(22) Date of filing: 18.06.2021
(51) Int. Cl.: A61K 36/66, A01P 1/00, A61L 15/32

(54) **DISINFECTANT SOLUTION AND BIODEGRADABLE DRESSING COMPRISING THE SAME**

(30) Priority: 24.02.2021 PT 2021117079
(71) Applicant: Instituto Politécnico De Leiria, 2411-901 Leiria (PT)
(72) Inventor: ANTUNES PAULINO, BRUNO FILIPE, 3405-306 LAJEOSA OHP (PT); VICENTE COSTA, CATARINA RAQUEL, 3420-457 VILA NOVA DE OLIVEIRINHA, TÁBUA (PT)
(74) Representative: Monteiro Alves, Inês

(57) **Abstract**

The present invention refers to a disinfectant solution comprising alcoholic extracts obtained from the plant *Chelidonium Majus L.,* wherein this is iodine-free and presents antibacterial and antifungal activity. A further object of the present invention is a biodegradable dressing which comprises a film and a cotton layer impregnated with the disinfectant solution.

The invention arise as an alternative to the iodine based disinfectant solutions currently available and provides biodegradable dressings which not only protect the wound, they also disinfect it.

## Description

### TECHNICAL FIELD

The present invention belongs to the field of medical sciences and refers to a disinfectant solution comprising alcoholic extracts obtained from the plant *Chelidonium Majus L..* A further object of the present invention is a biodegradable dressing which comprises a film and a cotton layer impregnated with the disinfectant solution.

### BACKGROUND ART

The celandine (*Chelidonium majus* L.) is a herbaceous and vivacious plant which is part of the poppy family. In Portugal it is also known as the "swallowwort", since it flowers in the beginning of spring and maintains the flower until fall, when the birds migrate.

Originating from Europe, the north of Africa and Occidental Asia, the celandine has its preferred habitat in places with rubble (ruderal species), over walls and fences (rupicolous species) or in fresh and shady soils.

The celandine exudes a yellow-orange sap, similar to iodine tincture, which is rich in carotenoids and toxic alkaloids and used in popular medicine to eliminate and treat warts, papilloma, condylomas and small skin surface lesions.

The flowering and dry parts, whole or fragmented, present, at least, 0,6% of total alkaloids, expressed in celandine. Apart from the benzophenanthridine core alkaloids (such as chelidonine, chelerythrine, sanguinarine), the celandine also has alkaloids from the protoberberine (berberine, coptisine) core, flavonoids, phenolic acids, resin and mucilage.

Physiological and therapeutic properties are attributed to some alkaloids of the *Chelidonium majus,* such as, for example, antimicrobial activity, analgesic and antiinflammatory effect (*European Medicines Agency, 2012*).

In view of the above, studies have been developed with the purpose of evaluating alternative therapeutics involving the extracts obtained from celandine due to the several properties thereof.

Prior art document EP2787958B1 describes pharmaceutical and cosmetic compositions based on celandine extracts for treating skin diseases and for skin regeneration.

The compositions described in this European document make use of the extracts obtained from the acid extraction of the aerial parts of *Chelidonium majus,* namely, flowers, flower buds, and leaves having no nerves. The extract obtained is submitted to a subsequent treatment step, such as dilution, concentration, sterilization, isolation of one or more components or supplementing with active substances of interest.

Korean document KR101980127B1 refers to a cosmetic composition for acne relief or for controlling oiliness containing a mixture of alkaloids from *Chelidonium majus* L. (such as chelidonine and chelerythrine) as active ingredient.

The composition described in this document does not use the celandine extract, however it uses the alkaloids that are present in the aerial parts thereof, namely, chelidonine and chelerythrine, or chemically synthetized alkaloids, in a concentration of up to 30% by weight.

If they are extracted from the celandine with an organic alcohol, the alkaloids of interest are separated by a chromatographic method and purified, for subsequent addition to the cosmetic composition.

Patent US10792322B2 proposes a pharmaceutical and/or phytosanitary composition comprising an aqueous extract of the root of the *Chelidonium majus* L. and excipients for preventing or treating fungal infections in the nails or in plants.

The composition described in the North-American document can further comprise an aqueous extract of the leaf of the *Thuja occidentalis* and from around 1 to 5% of at least one natural phenolic compound.

In a distinct manner, the present invention describes a disinfectant solution comprising alcoholic extracts obtained from the plant *Chelidonium Majus L.,* particularly, from the stem of the referred plant, as well as a biodegradable dressing which comprises a film and a cotton layer impregnated with the disinfectant solution.

The invention arise as an alternative to the iodine based disinfectant solutions that are currently available and provides biodegradable dressings which not only protect the wound, they also disinfect it.

### TECHNICAL PROBLEMS

Most of the studies and publications which refer to compositions based on extracts of *Chelidonium Majus L.* describe extracts obtained from the flowers, buds, leaves or roots of the referred plant.

Additionally, in some cases, it is preferred that the alkaloids of interest be separated from the extraction solvent, and then incorporated in the compositions, which makes it necessary to additionally process the extract for separation and purification of the components.

On the other hand, the present invention describes a disinfectant solution which comprises alcoholic extracts obtained from the stem of the *Chelidonium Majus L.,* wherein the referred extract is associated to the remaining components without the need for additional processing.

Further, the present invention also provides a biodegradable dressing which comprises a film and a cotton layer impregnated with the disinfectant solution, which meets the environmental issue raised by the use of quick dressings that are currently commercialized for the protection of the skin during treatment.

Further, the disinfectant solution of the present invention presents iodine in its constitution, which, in excess, is harmful to the human beings and can pollute the water resources when discarded inadequately.

### SUMMARY OF INVENTION

The present invention refers, in a first aspect, to a disinfectant solution comprising:
- 3.8 to 6.3 % by volume of an alcoholic extract obtained from the stem of *Chelidonium majus* L., with a concentration from 30 to 50 mg mL⁻¹ of celandine;
- 1 to 3 % by volume of an emollient agent selected from glycerin and sorbitol and mixtures thereof;
- 0.2 to 0.4 % by volume of an antioxidant agent selected from alpha tocopherol, ascorbic acid or polyphenols extracted from tuber peels and mixtures thereof; and
- water in a quantity sufficient to reach 100 %;
wherein the solvent of the alcoholic extract is ethanol, isopropanol or mixtures thereof.

The invention further describes, in a second aspect, biodegradable dressings which comprise a film and a cotton layer impregnated with the disinfectant solution, wherein the referred film comprises:
- 15 to 20 % by mass, in relation to the film forming dispersion, of an aqueous extract of *Chelidonium majus* L. with a concentration from 150 to 200 mg mL⁻¹ of celandine;
- 1 to 5 % by mass, in relation to the film forming dispersion, of starch extracted from tubers or from the wash water from corn, rice and flour-based pasta;
- 1 to 30 % by mass, in relation to the starch mass, of an emollient agent selected from the group consisting of glycerin or sorbitol and mixtures thereof;
- 2 to 3 % by mass, in relation to the starch mass, of a film forming agent selected from the group consisting of commercial guar, gelatin or guar gum and mixtures thereof;
- 0.3 to 1 % by mass, in relation to the starch mass, of an antioxidant agent selected from the group consisting of alpha tocopherol, ascorbic acid or polyphenols extracted from tuber peels and mixtures thereof; and
- a weak acid in an amount sufficient to correct the pH of the solution in a range varying from 2 to 5, preferably 4.

### SOLUTION TO PROBLEM

The present invention solves several state-of-the-art problems related to the therapeutic use of the celandine extracts.

The disinfectant solution now proposed presents antiseptic properties and is iodine-free, which compound, in excess, is harmful to human beings and is a pollutant of water resources.

Further, a biodegradable dressing is also proposed which comprises a film and a cotton layer impregnated with the disinfectant solution, wherein the referred dressing has a double function: protection and disinfection.

### ADVANTAGEOUS EFFECTS OF INVENTION

One of the main advantages presented by the present invention resides in the fact that the disinfectant solution now described does not present iodine in its composition. Iodine, although it is essential for the production of hormones by the thyroid gland, is harmful to the human being when in excess. Further, it can also be a pollutant of the water resources if it is improperly discarded.

Further, the biodegradable dressings proposed not only protect the wound, they also disinfect it, thus presenting a double functionality.

In this manner, the present invention is an alternative to the disinfectant solutions and dressings that are currently available.

### DESCRIPTION OF EMBODIMENTS

The invention refers, in a first aspect, to a disinfectant solution comprising extracts obtained from *Chelidonium Majus* L..

In a preferred embodiment of the invention, the extracts are alcoholic extracts obtained from the stem of the *Chelidonium Majus* L..

The referred solution therefore comprises:
- 3.8 to 6.3 % by volume of an alcoholic extract obtained from the stem of *Chelidonium Majus* L., with a concentration from 30 to 50 mg mL⁻¹ of celandine, preferably 5% of alcoholic extract of celandine at 40 mg mL⁻¹;
- 1 to 3 % by volume of an emollient agent selected from glycerin and sorbitol and mixtures thereof, preferably 2% of glycerin;
- 0.2 to 0.4 % by volume of an antioxidant agent selected from alpha tocopherol, ascorbic acid or polyphenols extracted from tuber peels (such as potato) and mixtures thereof, preferably 0.3 % of alpha tocopherol; and
- water in an amount sufficient to reach 100 %, wherein the water is distilled water or deionized water.

In one embodiment of the present invention, the alcoholic extract obtained from the celandine stem is ethanolic or isopropyl extract, obtained from a conventional extraction method which comprises the steps of:
a) drying of the plant species,
b) grinding of the dried plant species,
c) alcoholic extraction, and
d) filtration.

In step (a), the celandine stem is dried by means of a dehydration process in a hothouse with air circulation at temperatures from 40 to 60 °C, preferably 50 °C.

In step (b), the dried plant is ground to facilitate the action of the solvent. In a preferred embodiment, the grinding is carried out in a paddle mill.

Next, to prepare the alcoholic extracts of the plant, ethanol in a concentration that varies from 60 to 80% (v/v) was added to the dried and ground plant until a concentration from 30 to 50 mg mL-1 of celandine is obtained, preferably 40 mg mL-1 of celandine.

The alcoholic extraction is then carried out in step (c) at temperatures from 60 to 80 °C, preferably 70 °C, during time intervals that vary from 150 to 170 minutes, preferably 160 minutes, under stirring.

Finally, the extracts obtained are vacuum filtered.

In an alternative embodiment of the invention, the extracts are obtained by lyophilization followed by high pressure extraction.

Further, other ionic solvents can be used in the extraction process, such as, for example, the cetyl pyridinium chloride.

After obtaining the alcoholic extract, the disinfectant solution is reached by means of the addition of the remaining components and can be impregnated in a cotton layer adapted to a film to be applied as a biodegradable dressing.

For such, the film is obtained from the mixture of the following components:
- 15 to 20 % by mass, in relation to the film forming dispersion, of an aqueous extract of *Chelidonium majus* L. with a concentration from 150 to 200 mg mL⁻¹ of celandine, preferably 20% of the aqueous extract of *Chelidonium majus* L. with a concentration of 200 mg mL⁻¹ of celandine;
- 1 to 5 % by mass, in relation to the film forming dispersion, of starch extracted from tubers or from the wash water from corn, rice and flour-based pasta, preferably 1.2% by mass, in relation to the film forming dispersion, of starch extracted from potato;
- 1 to 30 % by mass, in relation to the starch mass, of an emollient agent selected from the group consisting of glycerin or sorbitol and mixtures thereof, preferably 20% by mass, in relation to the starch mass, of glycerin;
- 2 to 3 % by mass, in relation to the starch mass, of a film forming agent selected from the group consisting of commercial guar, gelatin or guar gum, preferably 2.5% by mass, in relation to the starch mass, of commercial agar;
- 0.3 to 1 % by mass, in relation to the starch mass, of an antioxidant agent selected from the group consisting of alpha tocopherol, ascorbic acid or polyphenols extracted from tuber peels, such as potato, and mixtures thereof, preferably 0.4 by mass, in relation to the starch mass, of alpha tocopherol; and
- a weak acid in an amount sufficient to correct the pH of the solution in a range varying from 2 to 5, preferably 4.

In a preferred embodiment of the present invention, the flour-based pasta corresponds to a mixture of flour of one or more cereals with water, wherein the cereals are selected from the group consisting of wheat, rye, barley, spelt and mixtures thereof.

In a preferred embodiment of the present invention, the weak acid is selected from acetic acid, citric acid and mixtures thereof.

The mixture is homogenized and heated under stirring until an increase in viscosity and polymerization is observed. The mixture is then transferred to a surface where it can be spread and subsequently dried.

After being dried, the film obtained is cut in the desired shape for the quick dressing. To this, a cotton layer is added, wherein the disinfectant solution of the present invention is impregnated.

Thus, the biodegradable dressing obtained according to the present invention presents antibacterial properties and disinfectant function.

### EXAMPLES OF THE INVENTION

### Example 1: Obtaining ethanoic extract from dried and raw celandine

The drying of the Chelidonium majus L. was carried out by the hothouse dehydration process with air circulation at 50 °C and, after the dehydration, the sample was ground in a paddle mill.

To prepare the ethanoic plant extracts, 2 grams of the sample were weighed and transferred to a test tube, next 50 mL of ethanol 80% (v/v) were added, obtaining a concentration of 40 mg mL-1 of celandine.

The extraction was carried out at 70 °C, in thermostatized water bath, for 30 minutes, under constant stirring. After this step the samples were filtered and used in the analyses of the antibacterial activity.

### Example 2: Analyses of antibacterial activity

### Preparation of the medium:

200g of chicken were cooked in 5L of water. The liquid was poured into an Erlenmeyer and left to cool in a fridge. The cooking water was filtered by means of a paper filter to a beaker. For each 100 mL of water 2g honey were added. The solution was neutralized (pH 7) with NaOH 0.1 mol L-1 and 4g agar were added for each 100 mL. This was autoclaved during 20 min at 120 °C and 1 atmosphere.

### Agar diffusion test:

For the evaluation of the antibacterial activity of the ethanoic extracts of Chelidonium majus L., the agar diffusion test was used, as taught by Blair et al. (1958).

For this purpose, there were applied 10 µL of the celandine extracts, in the in natura and dried form, which concentrations were of 133.0 mg mL-1 and 40 mg mL-1, respectively, in sterile Whatman No. 5 paper filters, disposed on Petri dishes, previously inoculated.

After the hothouse incubation at 37 °C for 24 hours, the antibacterial activity was determined by measuring the diameter of the inhibition zone (mm) around each disc.

In this test, the ethanol 80 % (v/v) was used as a negative control and the chloramphenicol antibiotic 80 µg mL-1 was used as positive control and the tests were carried out in triplicate.

At the end of the 24h, it was verified that both the raw and dried alcoholic extracts of the Chelidonium majus L. presented antibacterial activity, since there was an increase in the diameter of the inhibition zone. The same was not observed for the Chloramphenicol (positive control) and for the ethanol 80% (negative control).

### Example 3: Obtaining the biodegradable dressing.

### Extraction of the potato starch:

100g of old peeled potatoes were homogenized, in a liquefier, with 200mL of distilled water. This was filtered on a folded gauze, collecting the starch suspension in a 1L beaker. Let rest for 5 minutes. Disregard the supernatant and add 100mL of water, stir, let rest once more and leave it to settle. The procedure is repeated 10 times, and at the end, 200mL of distilled water are added and it was filtered in a Buchner filter and left to rest in a hothouse at 40° C for 72 h. The procedure was carried out in triplicate.

### Preparation of the aqueous celandine extract:

Around 30g of chopped Chelidonium majus L. were weighed and 150 mL of distilled water were added and heated until boiling, maintaining boiling for 15 minutes. This was left to rest for 10 minutes and filtered.

### Preparation of the film:

The preparation of the film was carried out in accordance with the method described by Almeida et al. (2014).

In a 150mL beacon 100mL of aqueous Chelidonium majus L. were added and 12g of extracted potato starch, 2mL glycerin and 2.24 g of commercial agar (20% of the starch amount), 0.05 g of alpha tocopherol and 2mL of acetic acid.

The reaction mixture was homogenized, followed by heating at 60 °C, under manual stirring, with help of a glass rod. After the increase in viscosity, the heating with agitation was maintained for around 5 minutes.

After the polymerization the mixture was transferred to a Petri dish, spread out in a film having around 1 centimeter thickness, being subsequently dried in a desiccator.

### Test for biodegradability on earth:

Three squares of the film obtained were cut, having dimensions of 2x2 cm and 0,1 mm thickness, and placed on Petri dishes containing fresh earth.

The plates were closed and sealed. The test was carried out in triplicate, being checked every 24 hours at room temperature. After 48h, fungi were identified.

The subject matter described above is provided as an illustration of the present invention and, therefore, cannot be interpreted so as to limit it. The terminology used herein with the purpose of describing preferred embodiments of the present invention must not restrict it to the same.

As used in the specification, the definite and indefinite articles, in their singular form, aim at the interpretation of also including the plural forms, unless the context of the description indicates, explicitly, the contrary. It will be understood that the expressions "comprise" and "include", when used in this description, specify the presence of the characteristics, the elements, the components, the steps and the related operations, however, they do not exclude the possibility of other characteristics, elements, components, steps and operations also being contemplated.

All the alterations, providing that they do not modify the essential characteristics of the claims that follow, must be considered as being within the scope of protection of the present invention.

### CITATION LIST

Follows the list of citations:

### PATENT LITERATURE

- EP2787958B1
- KR101980127B1
- US10792322B2

### NON PATENTARY LITERATURE

- Evaluation Report on Chelidonium majus L., European Medicines Agency (2012). Available at: https://www.ema.europa.eu/en/documents/herbal-report/final-assessment-report-chelidonium-majus-l-herba_en.pdf

## Claims

1. A disinfectant solution **characterized by** comprising:
- 3.8 to 6.3 % by volume of an alcoholic extract obtained from the stem of *Chelidonium majus* L., with a concentration from 30 to 50 mg mL⁻¹ of celandine;
- 1 to 3 % by volume of an emollient agent selected from glycerin and sorbitol and mixtures thereof;
- 0.2 to 0.4 % by volume of an antioxidant agent selected from alpha tocopherol, ascorbic acid or polyphenols extracted from tuber peels and mixtures thereof; and
- water in a quantity sufficient to reach 100 %;
wherein the solvent of the alcoholic extract is ethanol, isopropanol or mixtures thereof.

2. The disinfectant solution, according to claim 1, **characterized by** preferably comprising 5 % by volume of the alcoholic extract obtained from the stem of *Chelidonium majus* L. with a concentration of 40 mg mL⁻¹ of celandine.

3. The disinfectant solution, according to any one of the previous claims, **characterized by** preferably comprising 2 % by volume of the emollient agent.

4. The disinfectant solution, according to any one of the previous claims, **characterized by** the emollient agent being preferably glycerin.

5. The disinfectant solution, according to any one of the previous claims, **characterized by** preferably comprising 0.3 % by volume of the antioxidant agent.

6. The disinfectant solution, according to any one of the previous claims, **characterized by** the antioxidant agent being preferably alpha tocopherol.

7. The disinfectant solution, according to any one of the previous claims, **characterized by** the water being distilled water or deionized water, preferably distilled water.

8. A biodegradable dressing, **characterized by** comprising:
- a film comprising:
- 15 to 20 % by mass, in relation to the film forming dispersion, of an aqueous extract of *Chelidonium majus* L. with a concentration from 150 to 200 mg mL⁻¹ of celandine;
- 1 to 5 % by mass, in relation to the film forming dispersion, of starch extracted from tubers or from the wash water from corn, rice and flour-based pasta;
- 1 to 30 % by mass, in relation to the starch mass, of an emollient agent selected from the group consisting of glycerin or sorbitol and mixtures thereof;
- 2 to 3 % by mass, in relation to the starch mass, of a film forming agent selected from the group consisting of commercial guar, gelatin or guar gum and mixtures thereof;
- 0.3 to 1 % by mass, in relation to the starch mass, of an antioxidant agent selected from the group consisting of alpha tocopherol, ascorbic acid or polyphenols extracted from tuber peels and mixtures thereof; and
- a weak acid in an amount sufficient to correct the pH of the solution in a range varying from 2 to 5, preferably 4, and
- a cotton layer impregnated with the disinfectant solution as defined in any one of claims 1 to 7.

9. The biodegradable dressing, according to claim 8, **characterized by** preferably comprising 20 % by mass, in relation to the film forming dispersion, of aqueous *Chelidonium majus* L. extract with a concentration of 200 mg mL⁻¹ of celandine.

10. The biodegradable dressing, according to claim 8 or 9, **characterized by** preferably comprising 1.2 % by mass, in relation to the film forming dispersion, of starch.

11. The biodegradable dressing, according to claim 8 to 10, **characterized by** the starch being preferably extracted from potato.

12. The biodegradable dressing, according to claim 8 to 11, **characterized by** preferably comprising 20 % by mass, in relation to the starch mass, of emollient agent.

13. The biodegradable dressing, according to claim 8 to 12, **characterized by** the emollient agent being preferably glycerin.

14. The biodegradable dressing, according to claim 8 to 13, **characterized by** preferably comprising 2.5 % by mass, in relation to the starch mass, of film forming agent.

15. The biodegradable dressing, according to claim 8 to 14, **characterized by** the film forming agent being preferably commercial agar.

16. The biodegradable dressing, according to claim 8 to 15, **characterized by** preferably comprising 0.4 % by mass, in relation to the starch mass, of antioxidant agent.

17. The biodegradable dressing, according to any one of claims 8 to 16, **characterized by** the antioxidant agent being preferably alpha tocopherol.

18. The biodegradable dressing, according to any one of claims 8 to 17, **characterized by** the weak acid being selected from the group consisting of acetic acid, citric acid and mixtures thereof, preferably acetic acid.
